# EUROPEAN PATENT APPLICATION

(11) **EP 2 594 196 A1**
(43) Date of publication of application: **22.05.2013**
(21) Application number: 12250172.9
(22) Date of filing: 15.11.2012
(51) Int. Cl.: A61B 5/0488, A61F 2/72, A61B 5/1455

(54) **A sensor arrangement for detecting muscle activity for the control of technical equipment**

(30) Priority: 16.11.2011 DE 102011118732
(71) Applicant: Rslsteeper Group Limited, Kent ME2 4DP (GB)
(72) Inventor: Buchenrieder, Klaus, 85521 Riemerling (DE); Herrmann, Stefan, 82008 Unterhaching (DE)
(74) Representative: Crouch, David John

(57) **Abstract**

A sensor arrangement for detecting muscle activity for the control of technical equipment. When in use the arrangement covers a region of the skin surface of a user, to provide a signal indicative of muscle activity in a limited region for subsequent processing. The arrangement has at least one double-differential myoelectric sensor (1, 2, 3, 13, 14, 17, 18), together with at least one near-infrared sensor (4, 5, 9, 10, 12, 19, 20). These are for simultaneous or time-delayed derivation of (a) myoelectric activity and (b) the value of a parameter of the blood (for example the blood oxygen content or the relative quantity of haemoglobin) respectively, in the muscle, the muscles or the tissue under the arrangement.

## Description

The present invention relates to a sensor arrangement for detecting muscle activity for the control of technical equipment such as, for example, a hand or a leg prosthesis, or a computer or other device.

It has already been proposed to arrange the electrodes of myoelectric sensors on the skin surface close to muscles, the sensors detecting part of the depolarisation signal emitted in all directions and enabling further processing of the detected signal [LUCA, ALTER, and HERR09 of the references listed herein]. It is furthermore known that the depolarisation signals from simultaneously stimulated, innervated muscles are overlaid. If it is desired to measure signals from activity of a particular muscle or in a particular muscle region, this can result in cross-talk contamination. This effect increases with a growing spacing of such electrodes and is more pronounced in myoelectric sensors with single-differential electrodes than in sensors with double-differential electrodes [FARINA, DELSYS].

It has also been proposed to use near-infrared spectroscopy (NIRS), an active measuring method for the relative measurement of the blood oxygen content, with at least one light transmitter and one receiver [ASLIN, CHARM], to investigate muscle activity [AKIMA, QUARE, MIURA01, MIURA03].

The combination of a single-differential myoelectric sensor with a simple near-infrared sensor, consisting of a light source and a photo receiver to detect muscle activity, has also been proposed [HERR10A, HERR10B, HERR11A, HERR11B]. It has also been proposed to combine signals of the two sensors synchronously with a suitable weighting. A detailed discussion about the weighted combination of two features in one-time window has also been put forward [HERR10C].

With previously proposed combinations of a myoelectric and near-infrared sensor it is noted that it is only possible to detect one region under the sensor, which cannot be varied during the measurement. The overlays of the myoelectric signals of a plurality of muscles are recorded at a recording point and allow the basic distinction between different contraction types. However, there is no information about the spatial origin of the individual signal components. A near-infrared sensor, in contrast to this, only detects a spatially narrowly limited region below the sensor. This sensor type only indirectly allows an inference of muscle activity, as the blood quantity, and therefore the quantity of the haemoglobin, is influenced by a contraction.

The problem to be addressed is the derivation of a control signal that is generally proportional to the muscle contraction, enabling a statement about a spatially narrowly limited, well definable region in the musculature with minimal interferences or cross-talk and without signal artefacts or artificially induced error. The near-infrared signal with a high spatial resolution cannot be successfully used on its own to control technical equipment such as a prosthesis, because the relationship between the muscle force exerted and the measured signal amplitude is not sufficiently proportional.

This problem is addressed in accordance with the present invention by providing a sensor arrangement for detecting muscle activity for the control of technical equipment, which arrangement when in use covers a region of the skin surface of a user, to provide a signal indicative of muscle activity in a limited region for subsequent processing, in which the arrangement is provided with at least one double-differential myoelectric sensor, together with at least one near-infrared sensor for simultaneous or time-delayed derivation of (a) myoelectric activity and (b) the value of a parameter of the blood (for example the blood oxygen content, or the relative quantity of haemoglobin) respectively, in the muscle, the muscles or the tissue under the arrangement.

The blood oxygen content may be determined by means of the light intensity, as explained in the listed references, and contraction inferred. The penetration depth of the light into the tissue, and therefore the observation depth, is determined by the spacing apart of the NIR transmitter and the NIR receiver. As different movements have characteristic time-variant patterns in the electromyographic (EMG) and NIR signal, the movement can be inferred by measuring the two signals and thus, for example, an arm prosthesis can be controlled successfully and in a realistic manner.

The control signals for an arm, hand or leg prosthesis, for example, are produced by fusing the sensor signals and by combining the sensors. The myoelectric signal is used here for the general recognition of the beginning of a contraction. Following this in terms of time, in a short time period, the muscle contraction is classified within a spatially limited region by means of an NIR signal. When using a plurality of NIR sensors, the reliability of the classification and moreover also muscle activities in different muscle regions are distinguished and classified. Following the classification phase, which is short in terms of time, and movement or action of technical equipment, for example a prosthesis, can be carried out depending upon the myoelectric signal.

The present invention extends to a method of detecting muscle activity for the control of technical equipment using a sensor arrangement in accordance with the present invention.

Examples of sensor arrangements made in accordance with the present invention will now be described in greater detail with reference to the accompanying drawings, in which:

- Figures 1 to 5: show respective different configurations of sensor parts of sensor arrangements embodying the present invention; and

- Figures 6a and 6b: show respective graphs demonstrating operation of sensor parts shown in Figures 1 to 5.

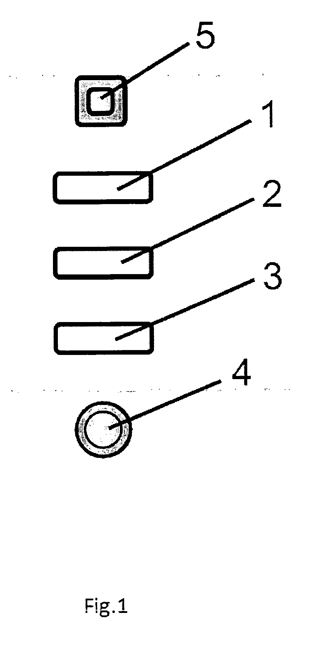

The configurations shown in Figures 1 to 5 comprise myoelectric sensor (EMG) electrodes, NIR transmitters and NIR receivers in respective different spatial arrangements. In every case, they are mounted on a mound of plastics material (not shown) so as to protrude inwardly from a skin covering (not shown) worn by the user, in such a fashion as to be in contact with the skin of the user. The relative spatial arrangements result in precisely defined regions in the tissue being observed. Thus the Figures in each case show the support face of the arrangement from the skin side. Electronic circuitry (not shown) is housed within the mounds, and is connected between the electrodes, transmitters and receivers on the one hand, and control outputs (not shown) on the other.

Figure 1 shows centrally arranged double-differential EMG sensor electrode pairs 1 and 2, and 2 and 3, with which the myoelectric activity of the muscles located therebelow is detected. The electrodes of the EMG sensor consist of three conductive areas 1, 2 and 3. These are bare metal contacts with downstream instrument amplifiers (not shown) of the INA 121 type from the company Texas Instruments. A near-infrared transmitter 4, comprising an ultra-bright source, such as, for example, a light-emitting diode or light-emitting diode arrangement in an encapsulated housing, of the type L4*730/4*805/4*850-40Q96-1 of the producer Epitex Incorporation is provided. A sensitive near-infrared receiver 5, for example a photodiode or a photo transistor with an amplifier connected downstream or integrated in a housing, of the OPT101 type from the producer Texas Instruments, is also provided. The transmitter/receiver pair 4/5 constitute parts of an NIR sensor of the embodiment. The EMG sensor electrodes 1, 2 and 3 are located between the transmitter 4 and the receiver 5, and the transmitter 4, electrodes 1, 2 and 3 and the receiver 5 are uniformly spaced and are arranged linearly, with their respective centres lying on the same imaginary straight line.

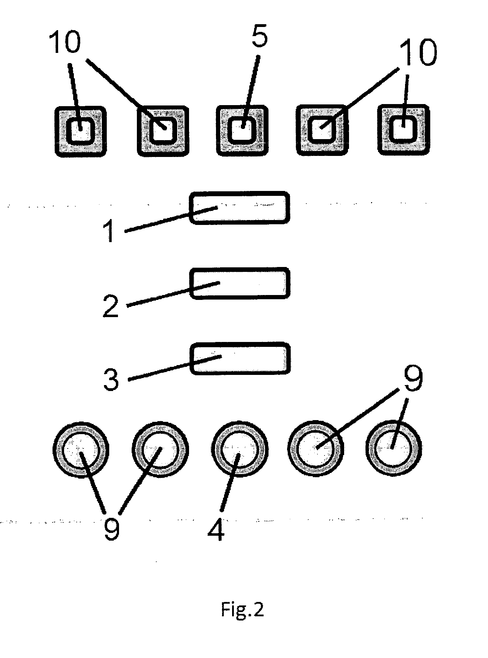

The configuration shown in Figure 2 is the same as that shown in Figure 1, but with two additional NIR transmitters 9 on one side of the transmitter 4, and two additional NIR transmitters 9 on the other side thereof, so that there are five transmitters 4, 9 altogether arranged with their centres lying on an imaginary line at right angles to the imaginary line on which the electrodes 1, 2 and 3 lie. In a corresponding fashion, the configuration shown in Figure 2 also has four additional NIR receivers 10. Thus the transmitters and receivers are arranged next to the EMG sensor electrodes such that they can provide an indication of the blood parameter, for example the blood oxygen content, or the relative quantity of the haemoglobin, in the muscle and the tissue and the blood vessels at a depth along the muscle and slightly obliquely along the muscle.

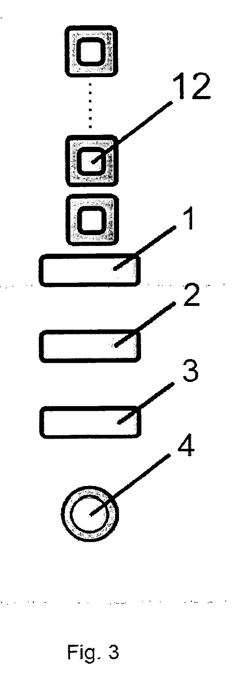

In the embodiment of Figure 3 the single NIR receiver 5 of the Figure 1 configuration has been replaced by a number of NIR receivers 12 spaced apart linearly with their centres lying on the same imaginary line is that on which lie the centres of the EMG electrodes 1, 2 and 3. The spacing between the receivers 12 is less than that between the electrodes 1, 2 and 3. When in use with the transmitter 4 illuminated, the non-absorbed light arriving at the different receivers 12 provides a measure of a blood parameter at different respective depths under the EMG electrodes 1, 2 and 3. The signals from the receivers 12 may be delivered to a central processor (not shown) of the arrangement simultaneously or consecutively.

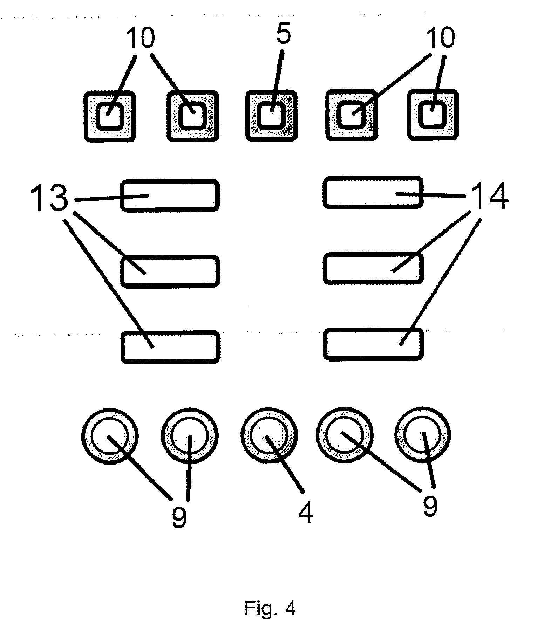

In the embodiment shown in Figure 4, the EMG electrodes 1, 2 and 3 of the Figure 2 arrangement have been replaced by two spaced columns of EMG electrodes 13 and 14 extending symmetrically between the rows of transmitters 4, 9 and receivers 5, 10.

The two columns of EMG electrodes 13 and 14 allow the myoelectric signals of a large muscle, for example on the leg, or a plurality of muscles located next to one another, for example in the chest region, to be detected simultaneously. The signals of the electrodes 13 and 14 are used here either individually or as correlated signals, for example to reduce the noise or remove interferences or artefacts.

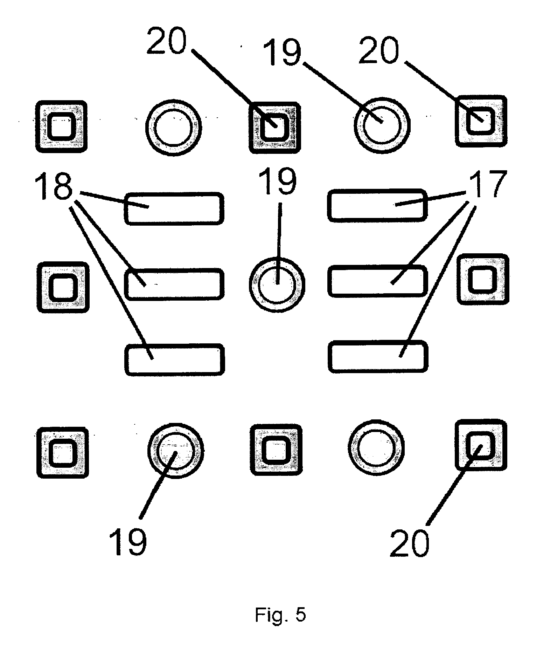

The embodiment shown in Figure 5 comprises two spaced columns 17 and 18 of EMG electrodes extending between two rows of NIR devices, each row comprising three NIR receivers 20, a first transmitter 19 between the middle NIR receiver 20 of the row and an outer receiver 20 thereof, and a second NIR transmitter 19 between the middle NIR receiver 20 of the row and the other outer receiver 20. A further transmitter 19 is located midway between the columns 17 and 18 and the rows of NIR devices. A further NIR receiver 20 is located between the end devices on one side of the rows, and another between the end devices on the other side of the rows. This array of EMG electrodes and NIR devices allows the blood oxygen content, or the relative haemoglobin quantity, to be measured at different depths and regions either simultaneously or consecutively.

In all the illustrated configurations, the EMG and the NIR signals are not evaluated simultaneously, but consecutively. The reason for this is the different signal properties and the information contained therein which are based on various underlying physical effects. The mean value of myoelectric signal is approximately proportional to the muscle force exerted. However, inferences about the type of contraction exerted can hardly be drawn from a single myoelectric signal, as an overlay of signals of a plurality of muscles occurs, so that it is not possible to determine the origin merely from the myoelectric signal. If, as, for example, in the forarm, a plurality of muscles are located in the surroundings of an EMG electrode, a contraction signal may be detected, but the distinction cannot be made as to which one has carried out a contraction. Owing to the fixed and spatially delimited observation region of an NIR sensor, contractions of the different muscles can be resolved with the aid of such a sensor. However, the resulting NIR signal is not proportional to the force developed and also only applies meaningful information at the beginning of a contraction. For this reason, in each of the illustrated embodiments of the present invention, its electronic circuitry (not shown) is so constructed or programmed that the EMG signal is firstly used for recognition as to whether a muscle contraction is present at all. The NIR signal cannot be used for this, as it is subject to interferences due to pressure changes or changes in the surrounding light. Once a contraction has been recognised, the NIR signal over a short period of time can be used to classify the type of contraction. As no inferences as to the strength of the contraction are possible by this, however, the EMG signal is used again following the classification in order to control movement proportionally, in other words depending upon the muscle force exerted.

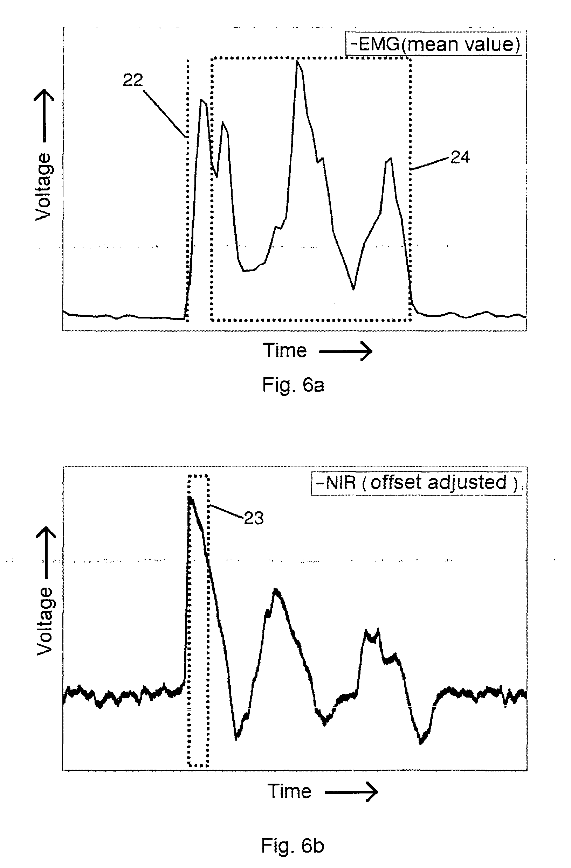

Figures 6a and 6b show derived EMG and NIR signals for a hand movement over the same time period. Once the EMG signal has exceeded a predefined threshold value at time 22, the type of movement is indicated by the NIR signal in the time window 23 for classification. The intensity of the movement is then determined by the course of the EMG signal amplitude in the time window 24. The movement and force control, for example of a hand prosthesis, is determined therefrom by calculation by known control engineering algorithms.

### Literature references:

- [ALTER]: Alter, Ralph (1966), Bioelectric Control of Prostheses, Technical Report: Massachusetts Institute of Technology, Research Laboratory of Electronics
- [AKIMA]: Akima, H. (2005), Functional Imaging of Human Skeletal Muscle During Movement: Implications for Recruitment, Metabolism and Circulation. International Journal of Sport and Health Science, Vol. 3, pp. 194-207.
- [ASLIN]: Aslin, R. and Mehler J. (2005), Near-infrared spectroscopy for functional studies of brain activity in human infants: promise, prospects and challenges. Journal of Biomedical Optics, Vol. 10. pp. 011009.
- [CHALM]: Chalmers, J. and Griffiths; P. eds. (2001), Handbook of Vibrational Spectroscopy. John Wiley & Sons.
- [DELSYS]: De Luca, C.J. (2007), A Practicum on the Use of sEMG Signals in Movement Sciences. Delsys Inc.
- [FARINA]: Farina, D., Merletti, R., Indino, B., Nazzaro, M. and Pozzo, M. (2002), Surface EMG crosstalk between knee extensor muscles: Experimental and model results. Muscle & Nerve, 26, pp. 681-695.
- [HERR09]: Herrmann, S. and Buchenrieder, K. (2009), Effects of Muscle Fatigue on Myoelectric Signal and Features in the Time-Domain (2009). African Journal of Information and Communication Technology. Eingereicht 2009.
- [HERR10A]: Herrmann, S. and Buchenrieder, K. (2010), Advanced Control Schemes for Upper Limb Prostheses. Advances in Medicine and Biology, Nova Science Publishers, Vol. 15, Chapter 17.
- [HERR10B]: Herrmann, S. and Buchenrieder, K. (2010), Development of a Combined Myoelectric and Near-infrared Sensor for Prostheses Control (2010). Proceedings of the 7th IASTED International Conference on Biomedical Engineering BioMed 2010, pp. 181-187.
- [HERR10C]: Herrmann, S. and Buchenrieder, K. (2010), Fusion of Myoelectric and Near-infrared Signals for Prostheses Control. Proceedings of the 4th International Convention on Rehabilitation Engineering & Assistive Technology, Article No: 54.
- [HERR11A]: Herrmann, S., Attenberger, A. and Buchenrieder, K. (2011), Prostheses Control with Combined Near-infrared and Myoelectric Signals. EUROCAST 2011, Part II, LNCS 6928, pp. 602-609.
- [HERR11B]: Herrmann, S. (2011), Direkte und proportionale Ansteuerung einzelmer Finger von Handprothesen. Dissertation, University of the Federal Armed Forces, Munich, Faculty of Information Technology, Graduation date: 25. November 2011.
- [LUCA]: De Luca, Carlo (2006), Electromyography. Encyclopedia of Medical Devices and Instrumentation, John Wiley Publisher, pp. 98-109
- [MIURA01]: Miura, H., McCully, K., Hong, L., Nioka, S. and Chance, B. (2001), Regional Difference of Muscle Oxygen Saturation and Blood Volume during Exercise Determined by Near Infrared Imaging Device. Japanese Journal of Physiology, Vol. 51, pp. 599-606.
- [MIURA03]: Miura, H., McCully, K. and Chance, B. (2003), Application of Multiple NIRS imaging device to the exercising muscle metabolism. Spectroscopy, Vol. 17, pp. 549-558.
- [QUARE]: Quaresima, V., Colier, W., van der Sluijs, M., and Ferrari (2001), Nonuniform Quadriceps O2 Consumption Revealed by Near Infrared Multipoint Measurements. Biochemical and Biophysical Research Communications, Vol. 285, pp. 1034-1039.

## Claims

1. A sensor arrangement for detecting muscle activity for the control of technical equipment, which arrangement when in use covers a region of the skin surface of a user, to provide a signal indicative of muscle activity in a limited region for subsequent processing, **characterised by** at least one double-differential myoelectric sensor (1, 2, 3, 13, 14, 17, 18) together with at least one near-infrared sensor (4, 5, 9, 10, 12, 19, 20) for simultaneous or time-delayed derivation of (a) myoelectric activity and (b) the value of a parameter of the blood (for example the blood oxygen content or the relative quantity of haemoglobin) respectively, in the muscle, the muscles or the tissue under the arrangement.

2. A sensor arrangement according to claim 1, **characterised in that** the said at least one near-infrared sensor (4, 5, 9, 10, 12, 19, 20) comprises a near-infrared transmitter (4, 9, 19) and a plurality of near-infrared receivers (5, 10, 12, 20), and **in that** an electrode (1, 2, 3, 13, 14, 17, 18) of the said at least one double-differential myoelectric sensor (1, 2, 3, 13, 14, 17, 18), the near-infrared transmitter (4, 9, 19) and the plurality of near-infrared receivers (5, 10, 12, 20), are arranged linearly, in an array, in an arcuate configuration or in a circular configuration, to enable measurement of a parameter of the blood, such as the blood oxygen content or the relative quantity of haemoglobin, in different depths, layers or regions from which myoelectric signals are detected by the said electrode (1, 2, 3, 13, 14, 17, 18).

3. A sensor arrangement according to claim 1, **characterised in that** the said at least one near-infrared sensor (4, 5, 9, 10, 12, 19, 20) comprises a plurality of near-infrared transmitters (4, 9, 19) and a near-infrared receiver (5, 10, 12, 20), and **in that** an electrode (1, 2, 3, 13, 14, 17, 18) of the said at least one double-differential myoelectric sensor (1, 2, 3, 13, 14, 17, 18), the plurality of near-infrared transmitters (4, 9, 19) and the near infrared receiver (5, 10, 12, 20), are arranged linearly, in an array, in an arcuate configuration or in a circular configuration, to enable a measurement of a parameter of the blood, such as the blood oxygen content or the relative quantity of haemoglobin, in different depths, layers or regions from which myoelectric signals are detected by the said electrode (1, 2, 3, 13, 14, 17, 18).

4. A sensor arrangement according to claim 1, **characterised in that** the said at least one near-infrared sensor (4, 5, 9, 10, 12, 19, 20) comprises one or more near-infrared transmitters (4, 9, 19) and one or more near-infrared receivers (5, 10, 12, 20), and **in that** an electrode (1, 2, 3, 13, 14, 17, 18) of the said at least one double-differential myoelectric sensor (1, 2, 3, 13, 14, 17, 18), the said one or more near-infrared transmitters (4, 9, 19) and the said one or more near-infrared receivers (5, 10, 12, 20), are arranged linearly, in an array, in an arcuate configuration or in a circular configuration, to enable a measurement of a parameter of the blood, such as the blood oxygen content or the relative quantity of haemoglobin, in different depths, layers or regions from which myoelectric signals are detected by the said electrode (1, 2, 3, 13, 14, 17, 18).

5. A sensor arrangement according to any preceding claim, **characterised in that** one or more electrodes (1, 2, 3, 13, 14, 17, 18) of a myoelectric sensor (1, 2, 3, 13, 14, 17, 18) of the arrangement, or one or more near-infrared transmitters (4, 9, 19) or one or more near-infrared receivers (5, 10, 12, 20) of the near-infrared sensor (4, 5, 9, 10, 12, 19, 20) are operated in pulsed mode, the pulses of which operation overlap or to not overlap as a function of time, in order to decouple signals, or to couple them, or to keep the mutual influencing of the signals low, or to exclude it, or to save energy.

6. A sensor arrangement according to any preceding claim, **characterised in that** signals indicative of the derived myoelectric activity and the value of parameter of the blood are not evaluated simultaneously, but consecutively, wherein -in the rest state, in which no muscle activity is present, the beginning of a contraction, as indicated by a threshold value of the signal indicative of myoelectric activity being exceeded, is awaited, and thereupon -in the movement state, in which muscle activity is present, the signal from the at least one near-infrared sensor(4, 5, 9, 10, 12, 19, 20) indicative of the value of a parameter of the blood is used to classify the movement, the contraction or the body part position.

7. A sensor arrangement according to claim 6, **characterised in that** following the classification, a signal from the said at least one double-differential myoelectric sensor (1, 2, 3, 13 14, 17, 18) is used to calculate or obtain for the subsequent processing, a control signal, which is generally proportional to the muscle force exerted.

8. A method of detecting muscle activity for the control of technical equipment using a sensor arrangement as claimed in any preceding claim.
